# EUROPEAN PATENT APPLICATION

(11) **EP 4 768 121 A1**
(43) Date of publication of application: **01.07.2026**
(21) Application number: 23952940.7
(22) Date of filing: 26.12.2023
(51) Int. Cl.: B01J 29/03, B01J 29/08, B01J 29/16, B01J 29/18, B01J 29/26, B01J 29/40, B01J 29/48, B01J 29/70, B01J 29/78, B01J 37/06, B01J 37/08, C07D 301/08, C07D 303/04

(54) **CATALYST FOR EPOXIDATION OF PROPYLENE WITH OXYGEN TO PREPARE PROPYLENE OXIDE AND USE THEREOF**

(30) Priority: 18.09.2023 CN 202311199882
(71) Applicant: Nankai University, Tianjin 300071 (CN)
(72) Inventor: LI, Landong, Tianjin 300071 (CN); LI, Weijie, Tianjin 300071 (CN); WU, Guangjun, Tianjin 300071 (CN)
(74) Representative: Wang, Bo
(86) International application number: PCT/CN2023/141940
(87) International publication number: WO 2025/060297

(57) **Abstract**

The present disclosure provides a catalyst for preparation of propylene oxide by epoxidation of propylene with oxygen, and use thereof. The catalyst comprises a zeolite support and an active component which is at least one of titanium, tin, molybdenum, or tungsten. A preparation method of the catalyst comprises: subjecting a silica-alumina zeolite to a dealumination treatment, mixing it with an organometallic compound precursor, and calcining the mixture in air. The present disclosure further provides use of the catalyst for direct epoxidation of propylene with oxygen to produce propylene oxide, and can solve problems in current propylene oxide synthesis, such as the need for co-production, high explosion risk, environmental pollution, high energy consumption, and complicated process. The catalyst of the present disclosure exhibits high propylene conversion and propylene oxide selectivity, and maintains favorable stability and propylene oxide yield over a long period, making it suitable for large-scale industrial production.

## Description

### TECHNICAL FIELD

The present disclosure relates to the technical field of olefin epoxidation, and mainly relates to a catalyst for direct epoxidation of propylene with oxygen to produce propylene oxide and the use thereof.

### BACKGROUND

Propylene oxide (PO), as the third largest derivative of propylene, is a very important basic organic chemical raw material in the synthesis of downstream products and the production of fine chemicals. One of the most important roles of propylene oxide in the synthesis of downstream organic products is its use in the production of polyether polyols, which are essential raw materials for synthesizing polyurethanes. Furthermore, it is used as a surfactant. Propylene oxide is also a raw material for producing propylene glycol. Propylene glycol serves not only as a feedstock for the production of polyester resins, epoxy resins, and polyurethane resins but also as an intermediate in the manufacturing of surfactants. Additionally, it is used as a solvent in food flavors and fragrances as well as daily cosmetics. Propylene oxide has thousands of derivatives, which are widely used in thermal insulation materials for the construction industry, as well as in sectors including furniture, paints, elastomers, and adhesives for the processing and manufacturing industry. Given the important role of propylene oxide and the fact that the production capacity of propylene oxide in China is largely constrained by the level of synthesis technology and environmental protection policies, a huge production capacity gap of propylene oxide has consequently emerged.

The main preparation methods for propylene oxide include the chlorohydrin process, the indirect oxidation process, and the direct oxidation process. Among them, the indirect oxidation process includes the isobutane co-oxidation process (PO/TBA process), the ethylbenzene co-oxidation process (PO/SM process), and the cumene hydroperoxide process (CHPPO process). The direct oxidation process refers to the direct epoxidation reaction between propylene and an oxidant without the addition of other raw materials such as hydrogen. This comprises the liquid-phase epoxidation of propylene using liquid H₂O₂ or cumene hydroperoxide as the oxidant (HPPO); and the gas-phase direct epoxidation of propylene using molecular oxygen O₂ as the sole oxidant.

Except for the gas-phase direct epoxidation of propylene, which is still in the research and exploration stage, all the other methods mentioned above have been industrialized. Currently, the production capacity of propylene oxide (PO) via the chlorohydrin process accounts for 40% of the total capacity worldwide, while that via the indirect oxidation process makes up 43%, with the remaining 17% attributed to other production routes. However, these methods suffer from issues such as the need for co-production, high explosion risk, environmental pollution, high energy consumption, and process complexity.

Given its wide availability, low cost, and environmental benignity, using oxygen alone as the oxidant for the direct epoxidation of propylene is the most ideal approach. The direct epoxidation of propylene (DEP) with oxygen features 100% atom economy, along with the merits of environmental friendliness and sustainability. Therefore, the direct synthesis of propylene oxide from propylene and oxygen has become one of the most promising routes to be realized in the field of selective catalytic oxidation, as well as one of the most challenging reactions in the chemical industry.

However, at present, the direct epoxidation of propylene with oxygen for propylene oxide production has not yet been industrialized, owing to the difficulty in simultaneously achieving a considerable propylene conversion and high propylene oxide selectivity. The results reported so far have focused on maintaining high propylene oxide selectivity (>80%), while the propylene conversion can only reach around 2~3%, which is far from meeting the requirements for industrial production.

In the oxidation reaction of the gas-phase direct epoxidation of propylene with oxygen, the regulation of product selectivity proves difficult, and the development of catalysts has long been a major challenge. Furthermore, owing to the differences in reaction mechanisms, catalysts for the liquid-phase epoxidation process require hydrogen peroxide as the oxidant to form active Ti-OOH species for completing the epoxidation reaction. Therefore, catalysts for the liquid-phase process cannot be applied to the gas-phase epoxidation process.

In recent years, the inventors have carried out a series of studies on the direct epoxidation of olefins. Cobalt-based zeolite, reported in 2022, contains single isolated cobalt ion centers that can catalyze the combination of oxygen atoms from preferentially adsorbed oxygen molecules with two subsequently adsorbed propylene molecules to form two propylene oxide molecules, through a non-dissociative activation pathway. In this process, the completion of the Co²⁺-Co^{δ+}-Co²⁺ redox cycle enables the achievement of a considerable propylene conversion (~15%) with a certain propylene oxide selectivity (~50%) (J. Am. Chem. Soc., 2022, Vol. 144, pp. 4260~4268). However, this selectivity still falls short of the requirements for industrial scale-up.

Therefore, there is an urgent need to explore a catalyst for the gas-phase direct epoxidation of propylene to propylene oxide that can simultaneously improve both propylene conversion and propylene oxide selectivity.

### SUMMARY

To this end, an object of the present disclosure is to provide a catalyst for the gas-phase epoxidation of propylene to propylene oxide that can simultaneously improve both propylene conversion and propylene oxide selectivity.

To achieve the above object, the present disclosure provides a catalyst for direct epoxidation of propylene with oxygen to produce propylene oxide. The catalyst comprises a zeolite support and an active component, wherein the active component is at least one of titanium, tin, molybdenum, or tungsten. A preparation method of the catalyst comprises the following steps: (1) subjecting a silica-alumina zeolite to a dealumination treatment to obtain a treated zeolite; and (2) mixing the treated zeolite with a metal precursor for introducing an active component, and calcining the mixture in air to obtain the catalyst.

A dealumination degree of the zeolite support is 50~100%.

The active component is supported on the zeolite support.

A loading of the active component is 1~8% by mass of the catalyst.

In step (1), the silica-alumina zeolite comprises at least one of MOR, MFI, FAU, or BEA zeolite, and has a silicon-to-aluminum atomic ratio of 12~50.

The dealumination treatment described in the step (1) may be, for example, an acid dealumination treatment, specifically such as a concentrated acid heating reflux treatment, wherein the acid may be concentrated nitric acid or an organic acid. The temperature of the acid dealumination treatment is 60-120°C, and the duration is 5~50 hours. The degree of dealumination is determined by solid-state nuclear magnetic resonance (NMR) and energy-dispersive X-ray spectroscopy (EDS) analysis.

In step (1), the treated zeolite is a high-silica zeolite, the high-silica zeolite having a silicon-to-aluminum atomic ratio of greater than 12.

The step (1) comprises the following steps:
(1.1) calcining a silica-alumina zeolite raw powder (with an atomic ratio of n_{Si}/n_{A1} being 12~50) to remove a template, followed by heating a resulting product under acid treatment conditions at a temperature of 60~120°C for a duration of 5~50 hours to obtain a first material;
(1.2) cooling the first material, subjecting it to vacuum filtration using deionized water as the washing solvent; subsequently, drying a filter cake washed to neutrality in a forced-air drying oven at a temperature of 90~110°C for 12~24 hours to obtain a second material; and
(1.3) heating the second material obtained in step (1.2) to a temperature of 450~650°C at a heating rate of 2~5°C per minute and calcining it for 4~12 hours to obtain the treated zeolite.

The step (2) comprises the following steps:
(2.1) mixing the treated zeolite obtained in step (1) thoroughly with the metal precursor to obtain a mixture; and
(2.2) subsequently, calcining the obtained mixture in a furnace to obtain the catalyst.

In step (1.3) and/or step (2.2), the calcining may be performed in a muffle furnace.

In step (2), the temperature is raised to the calcination temperature at a heating rate of 2~5°C per minute, and the calcination temperature is 450~650°C and the calcination time is 4~12 hours.

In step (2), the mixing is carried out under an oxygen-free condition, wherein the oxygen-free condition comprises vacuum or an inert atmosphere.

In step (2), the mixing involves grinding the treated zeolite and the metal precursor uniformly in a mortar.

The metal precursor comprises at least one of an organometallic compound precursor and an inorganic metal compound precursor, preferably an organometallic compound precursor. The organometallic compound precursor comprises at least one of an organotitanium precursor, an organotin precursor, an organomolybdenum precursor, or an organotungsten precursor.

The organotitanium precursor comprises at least one of titanium acetylacetonate, bis(cyclopentadienyl)titanium dichloride, cyclopentadienyltitanium trichloride, tetraethyl titanate, tetraisopropyl titanate, tetrabutyl titanate, or tetrakis(2,4-pentanedionato)titanium(IV).

The organotin precursor comprises at least one of dimethyltin dichloride, tetramethyltin, or tin tetrachloride.

The organomolybdenum precursor comprises at least one of bis(cyclopentadienyl)molybdenum dichloride, bis(3-carbonylcyclopentadienyl)molybdenum, or molybdenum acetylacetonate.

The organotungsten precursor is tungsten hexacarbonyl.

The present disclosure further provides use of the above catalyst for direct epoxidation of propylene with oxygen to produce propylene oxide.

The present disclosure further provides a method for producing propylene oxide by direct epoxidation of propylene with oxygen, wherein the above catalyst is used to carry out the direct epoxidation of propylene with oxygen to produce propylene oxide.

The use of the catalyst for the direct epoxidation of propylene with oxygen to produce propylene oxide or the method for producing propylene oxide by the direct epoxidation of propylene with oxygen comprises:
(a) loading the shaped catalyst into a reactor, performing pretreatment and activation on the catalyst, then feeding a reaction gas comprising propylene, oxygen, and a balance gas, wherein the balance gas comprises an inert gas, the inert gas preferably being at least one of nitrogen, argon, or helium; and
(b) heating the reactor to 200~700°C and allowing the reaction to proceed for 1~3 hours until equilibrium is reached, thereby obtaining propylene oxide.

The reactor is a fixed-bed reactor operating at atmospheric or low pressure (lower than 2 MPa).

In step (a), the pretreatment and activation comprise pretreating and activating the catalyst with air at a temperature of 200~600°C for 1~3 hours.

In step (a), the reaction gas has a propylene volume concentration of 5~20% and an oxygen volume concentration of 2.5~12.5%.

In step (a), a space velocity for the reaction is 4,000~40,000/g _{catalyst}/hour.

The reaction process for direct epoxidation of propylene with oxygen provided by the present disclosure is as follows:

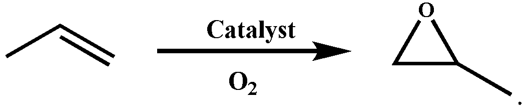

The method for efficient direct epoxidation of propylene with oxygen provided by the present disclosure is implemented by using the catalyst described herein, which is composed of titanium, tin, molybdenum, and tungsten as the main active components and a zeolite as the support, without the need for introducing other raw materials such as hydrogen. The catalyst is prepared by using a silica-alumina zeolite as a raw material, subjecting it to an acid dealumination treatment, and introducing active center metals through calcination. The method for preparing the catalyst involves mixing the dealuminated high-silica or pure-silica zeolite with metal precursors, followed by calcination in an air atmosphere. Through dealumination of the silica-alumina zeolite, residual aluminum in the catalyst that would adversely affect the yield and selectivity of propylene oxide product is avoided.

This catalyst differs from previous catalysts used for propylene oxide preparation in that it can utilize propylene and oxygen for direct oxidation without requiring additional additives, auxiliary gases, or hydrogen during the reaction process. The oxidant is oxygen obtainable directly from air, rather than other oxides (such as hydrogen peroxide, cumene hydroperoxide, etc.), thereby achieving a high propylene conversion and propylene oxide selectivity, and maintaining good stability and propylene oxide yield over long reaction periods, enabling large-scale industrial application.

The present disclosure employs a specific catalyst to achieve the preparation of propylene oxide by epoxidizing propylene using inexpensive oxygen as the sole oxidant, wherein the reaction pathway differs from that reported by the inventors of the present disclosure in the Journal of the American Chemical Society (2022, Vol. 144, pp. 4260-4268) using cobalt-based zeolite as catalyst to prepare propylene oxide. The reaction pathway of the present disclosure is analogous to chemical looping and the Mars-van Krevelen (MvK) mechanism, i.e., utilizing the active lattice oxygen adjacent to the metals including titanium, tin, molybdenum and tungsten for epoxidizing the substrate, and replenishing the oxygen vacancies with oxygen, thereby achieving efficient and selective epoxidation of propylene under the conditions of gas-phase fixed-bed reaction. In the process of this epoxidation reaction, the catalytic effect is exerted by the metal hydroxyl sites rather than the metal-OOH sites adopted previously when hydrogen peroxide was used as the oxidant. The reaction pathway for preparing propylene oxide using the catalyst provided by the present disclosure is different from that of the above cobalt-based zeolite, thereby enabling the present disclosure to achieve high propylene oxide selectivity (>80%) while attaining a propylene conversion that is much higher than the results reported previously (the propylene conversion of the propylene oxide preparation method of the present disclosure is >10%).

The preparation process of the present disclosure is simple and easy to operate with stable structural performance, and it can avoid the problems associated with the production of propylene oxide by other methods, such as the need for co-production, high explosion risk, environmental pollution, high energy consumption, and complicated process, thereby enabling large-scale industrial production of direct gas-phase epoxidation of olefins.

In light of the foregoing, the catalyst, the use of the catalyst, and the method for preparing propylene oxide via the gas-phase process using the catalyst provided by the present disclosure exhibit the following technical effects.

The inventors of the present disclosure have found that applying the catalyst of the present disclosure to the direct epoxidation of propylene with oxygen for propylene oxide production features a brand-new reaction mechanism and performance, enabling the simultaneous achievement of a high propylene conversion (>10%) and high propylene oxide selectivity (>80%). It also combines the advantages of direct propylene oxide production via the gas-phase process, featuring a simple and easy-to-operate synthesis method, relatively safe catalytic reaction conditions, and environmentally friendly and pollution-free reaction products. Thus, it can replace the existing propylene oxide preparation processes and be applied on a large scale.

Furthermore, the catalyst prepared by the present disclosure exhibits favorable reaction stability and coking resistance in the direct epoxidation of propylene with oxygen. In addition, by using zeolite as the catalyst support, the present disclosure significantly reduces the economic cost.

### BRIEF DESCRIPTION OF THE DRAWINGS

FIG. 1 shows an X-ray diffraction (XRD) pattern of a catalyst sample from Example 1.
FIG. 2 shows a scanning electron microscopy (SEM) image of a catalyst sample from Example 1.
FIG. 3 shows a transmission electron microscopy (TEM) image of a catalyst sample from Example 1.
FIG. 4 shows a stability evaluation diagram of a catalyst sample from Example 1 for the direct epoxidation of propylene.

### DETAILED DESCRIPTION

The present disclosure is described in further detail below with reference to specific examples. However, it should be understood that these examples are provided for illustration only and are not intended to limit the scope of the present disclosure. In addition, it should be understood that, after reading the teachings of the present disclosure, those skilled in the art can make various changes or modifications to the present disclosure, and these equivalent forms should also fall within the scope defined by the claims of the present disclosure.

According to the present disclosure, the dealumination degree of the zeolite catalyst can be varied within a certain range. Preferably, the dealumination degree of the catalyst ranges from 60% to 100%; more preferably, the dealumination degree of the catalyst ranges from 80% to 100%; and most preferably, the dealumination degree of the catalyst is 100%.

According to the present disclosure, the type of metal precursor in the catalyst may be varied within a certain range. For example, the titanium precursor in the catalyst may be either an organic precursor or an inorganic precursor, preferably an organic precursor, and more preferably selected from bis(cyclopentadienyl)titanium dichloride, titanium acetylacetonate, cyclopentadienyltitanium trichloride, tetraethyl titanate, tetraisopropyl titanate, tetrabutyl titanate, or tetrakis(2,4-pentanedionato)titanium(IV). The tin precursor in the catalyst is preferably an organic precursor, more preferably dimethyltin dichloride or tetramethyltin. The molybdenum precursor in the catalyst is preferably an organic precursor, more preferably bis(cyclopentadienyl)molybdenum dichloride, bis(3-carbonylcyclopentadienyl)molybdenum, or molybdenum acetylacetonate.

According to the present disclosure, a metal content in the catalyst, calculated on a metal element basis, may be varied over a relatively wide range. For example, a mass content of the metal in the catalyst, calculated on a metal element basis, ranges from 0.01% to 8%. Specific examples may include 0.01%, 0.05%, 0.1%, 0.2%, 0.3%, 0.4%, 0.5%, 0.6%, 0.7%, 0.8%, 0.9%, 1%, 1.2%, 1.4%, 1.6%, 1.8%, 2%, 2.5%, 3%, 3.5%, 4%, 5%, 6%, 7%, or 8%. Preferably, the metal content ranges from 0.2% to 5%, and more preferably from 1% to 4%.

According to the present disclosure, in the synthesis of the catalyst, a calcination temperature after mixing with the metal precursors ranges from 450°C to 650°C, preferably from 550°C to 650°C, and more preferably from 550°C to 600°C; a heating rate is more preferably 3~5°C/min; and a calcination time is more preferably 8~12 hours.

According to the present disclosure, a volume ratio of propylene to oxygen is preferably 2~0.5:1, and more preferably 0.8~1.2:1.

According to the present disclosure, generally, to ensure reaction safety, a volume concentration of propylene in the mixed gas shall not exceed 20%. For example, the volume concentration of propylene may be 5%, 6%, 7%, 8%, 9%, 10%, 12%, 14%, 16%, 18%, or 20%, preferably less than 16%, and more preferably less than 10%.

According to the present disclosure, generally, to ensure reaction safety, a volume concentration of oxygen in the mixed gas shall not exceed 12.5%. For example, the volume concentration of oxygen may be 2.5%, 3%, 4%, 5%, 6%, 7%, 8%, 9%, 10%, 11% or 12%, preferably higher than 3% and lower than 10%, and more preferably higher than 5% and lower than 10%.

According to the present disclosure, a temperature for direct epoxidation of propylene may be 200~700°C. However, to further improve the conversion, selectivity, and space-time yield of the reaction, as well as to extend a service life of the catalyst and reduce an amount of the catalyst used, a reaction temperature is preferably 200~600°C, and more preferably 300~600°C, with examples including 300°C, 340°C, 380°C, 420°C, 460°C, 500°C, 520°C, 540°C, 560°C, 580°C, and 600°C.

According to the present disclosure, the amount of the catalyst used may be varied within a relatively wide range. For example, the amount of the catalyst used may be 0.05~0.4 g, preferably 0.1~0.3 g, and more preferably 0.1 g~0.15 g.

According to the present disclosure, a space velocity for the propylene epoxidation reaction is 4,000~40,000/g _{catalyst}/hour. However, to further improve the conversion, selectivity, and space-time yield of the reaction, as well as to extend the service life of the catalyst and reduce the amount of the catalyst used, the space velocity for the reaction is preferably 8,000~40,000/g _{catalyst}/hour, and more preferably 16,000~36,000/g _{catalyst}/hour. For example, it may be 16,000/g _{catalyst}/hour, 18,000/g _{catalyst}/hour, 20,000/g _{catalyst}/hour, 22,000/g _{catalyst}/hour, 24,000/g _{catalyst}/hour, 26,000/g _{catalyst}/hour, 28,000/g _{catalyst}/hour, 30,000/g _{catalyst}/hour, 32,000/g _{catalyst}/hour, 34,000/g _{catalyst}/hour, or 36,000/g _{catalyst}/hour.

The specific experimental methods and equipment involved in the following examples and comparative examples are conventional methods or implemented based on the conditions recommended by the manufacturers, unless otherwise specified. All reagents involved are commercially available. A silicon-to-aluminum atomic ratio of a silica-alumina zeolite raw powder used therein is 12~50.

### Comparative Example 1:

A titanium-containing zeolite catalyst was prepared by an in-situ synthesis method different from that of the present disclosure and was used for the direct epoxidation of propylene with oxygen to produce propylene oxide.

0.74 g of tetrabutyl titanate was added to 20.8 g of tetraethyl orthosilicate solution cooled in an ice-water bath, followed by stirring for 2 hours. Then, 20.3 g of tetrapropylammonium hydroxide solution and 5.1 g of deionized water were added to the above mixture, and stirring was continued until a homogeneous mixture was obtained. The resulting final solution was transferred into a synthesis autoclave and subjected to high-temperature crystallization in an oven for 2~4 days. A solid obtained from the crystallization was subjected to suction filtration and washing until neutrality was reached, and then dried in an oven at 100°C for 12 hours. Finally, the solid was calcined in a muffle furnace at 550°C for 6~12 hours to obtain a catalyst used in Comparative Example 1, designated as Catalyst Ti-Ref. This catalyst was subsequently used for the direct epoxidation of propylene, and the specific results are shown in Table 1.

**Comparative Example 2:** A cobalt-containing zeolite catalyst was prepared by an in-situ synthesis method and used for the direct epoxidation of propylene with oxygen to produce propylene oxide (Journal of the American Chemical Society, 2022, Vol. 144, pp. 4260-4268).

1.05 g of cobalt nitrate hexahydrate was dissolved in 90 g of deionized water, and 1 mL of diethylenetriamine ligand was added dropwise, followed by stirring for 2 hours. Then, 1.5 g of sodium metaaluminate, 7.5 g of sodium hydroxide, and 11.5 mL of silica sol were respectively added to the above mixture, and stirring was continued until a homogeneous mixture was obtained. The resulting final solution was transferred into a synthesis autoclave and subjected to high-temperature crystallization in an oven for 2~4 days. A solid obtained from the crystallization was subjected to suction filtration and washing until neutrality was reached, and then dried in an oven at 100°C for 12 hours. Finally, the solid was calcined in a muffle furnace at 550°C for 6~12 hours to obtain a catalyst used in Comparative Example 2, designated as Catalyst Co-Ref. This catalyst was subsequently used for the direct epoxidation of propylene, and the specific results are shown in Table 2.

**Comparative Example 3:** A titanium-containing zeolite catalyst was prepared using a zeolite support different from that of the present disclosure and used for the direct epoxidation of propylene with oxygen to produce propylene oxide.

At room temperature, 0.4 g of MCM-41 zeolite was added to a chloroform solution containing 0.02 g of titanocene dichloride under argon protection. Subsequently, 1 mL of triethylamine was added, and the mixture was stirred for 2 hours until a color of the solution changed from red to yellow. The resulting solution was subjected to suction filtration and washing with chloroform three times, dried in an oven at 100°C for 12 hours, and finally calcined in a muffle furnace at 550°C for 6~12 hours, yielding the catalyst used for Comparative Example 1, designated as Catalyst Ti-MCM-41. This catalyst was subsequently employed for the direct epoxidation of propylene, with the specific results shown in Table 3.

**Example 1:** An aluminum-free catalyst was synthesized using bis(cyclopentadienyl)titanium dichloride as the organometallic compound precursor and used for the direct epoxidation of propylene with oxygen.
1) 10 g of a calcined silica-alumina zeolite raw powder was mixed with 200 mL of concentrated nitric acid in a three-necked flask, and the mixture was stirred and heated under reflux in an oil bath reactor at 100°C for 20 hours;
2) after the refluxed solid-liquid mixture was fully cooled, it was washed with deionized water and subjected to vacuum filtration until washings were neutral; the resulting filter cake was then placed in a forced-air drying oven at 100°C and dried at constant temperature for 12 hours; and the dried sample was characterized by solid-state NMR and EDS, confirming a dealumination degree of 100%;
3) the sample obtained in step 2) was calcined in a muffle furnace at 550°C for 12 hours with a heating rate of 2°C/min; and
4) 1.0 g of a solid powder obtained in step 3) and 0.15 g of the bis(cyclopentadienyl)titanium dichloride precursor were weighed out, ground and mixed thoroughly in a glove box, and then placed in a muffle furnace for high-temperature calcination, yielding a catalyst for Example 1, designated as Catalyst Ti-A, wherein the grinding time was more than 1 hour, the calcination temperature was 550°C, and the calcination time was 12 hours.

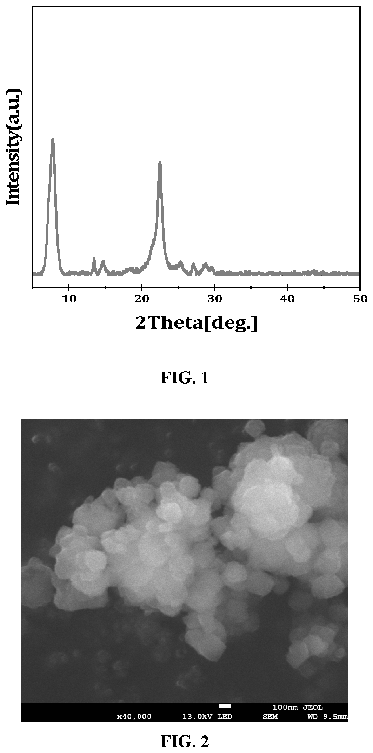

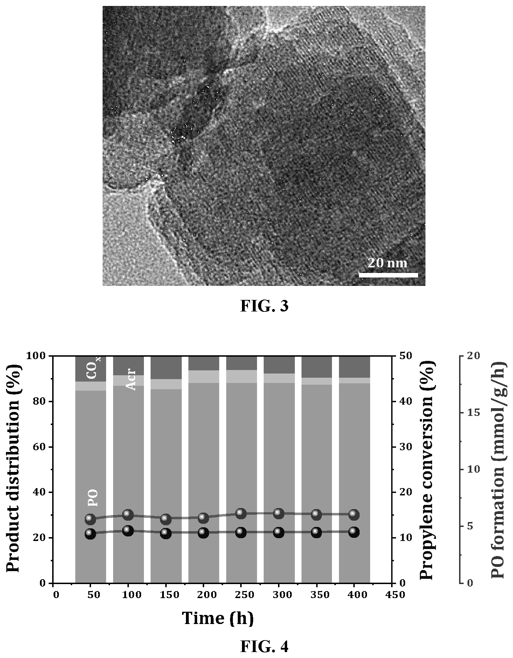

The characterization results of Catalyst Ti-A in Example 1 are shown in FIGS. 1, 2, and 3. FIG. 1 shows an XRD pattern, indicating that the sample possesses a standard microporous zeolite structure. An SEM image in FIG. 2 shows that the sample has a regular structure and size. A TEM image in FIG. 3 demonstrates that the sample contains well-ordered pore channels and exhibits good crystallinity.

Calculated from the characterization results, the dealumination degree of Catalyst Ti-A was 100%, and the mass content of titanium was 3%.

**Example 2:** An aluminum-containing catalyst was synthesized using bis(cyclopentadienyl)titanium dichloride as the organometallic compound precursor and used for the direct epoxidation of propylene with oxygen.

Example 2 is substantially the same as Example 1, except that the dealumination degree was controlled to be different from that of Example 1 by varying the reflux time in the oil bath. The resulting catalyst was designated as Catalyst TiAl-A.

Calculated from the characterization results, the dealumination degree of Catalyst TiAl-A was 65%, and the mass content of titanium was 3%.

**Examples 3~9**: Catalysts were synthesized using titanium acetylacetonate, cyclopentadienyltitanium trichloride, tetraethyl titanate, tetraisopropyl titanate, tetrabutyl titanate, and tetrakis(2,4-pentanedionato)titanium(IV) as the organometallic compound precursors, respectively. The other steps were the same as those in Example 1.

The obtained catalysts were designated as Catalyst Ti-B, Catalyst Ti-C, Catalyst Ti-D, Catalyst Ti-E, Catalyst Ti-F, Catalyst Ti-G, and Catalyst Ti-H, respectively.

**Examples 10~12:** Catalysts were synthesized using dimethyltin dichloride, tetramethyltin, and tin tetrachloride as the organometallic compound precursors, respectively. The other steps were the same as those in Example 1.

The obtained catalysts were designated as Catalyst Sn-A, Catalyst Sn-B, and Catalyst Sn-C, respectively.

**Examples 13~15:** Catalysts were synthesized using bis(cyclopentadienyl)molybdenum dichloride, bis(3-carbonylcyclopentadienyl)molybdenum, and molybdenum acetylacetonate as the organometallic compound precursors, respectively. The other steps were the same as those in Example 1.

The obtained catalysts were designated as Catalyst Mo-A, Catalyst Mo-B, and Catalyst Mo-C, respectively.

**Example 16:** A catalyst was synthesized using tungsten hexacarbonyl as the organometallic compound precursor. The other steps were the same as those in Example 1.

The obtained catalyst was designated as Catalyst W-A.

The catalysts obtained in the above examples and comparative examples were applied to the direct epoxidation of propylene with oxygen to produce propylene oxide, and their performance was evaluated. This process specifically comprised the following steps.

### (1) Sample Pretreatment and Activation

0.1 g of the shaped catalyst was loaded into an atmospheric pressure fixed-bed reactor, which was then subjected to pretreatment and activation with air at 300°C for 1 hour.

### (2) Direct Epoxidation of Propylene with Oxygen

A reaction gas was fed into the reactor in step (1), the reaction gas consisting of propylene, oxygen, and a balance gas, with a volume concentration of 5% for propylene, 5% for oxygen, a total gas flow rate of 60 mL/min, and a total space velocity of 36,000/hour; and the reactor was gradually heated from 300°C to 580°C to obtain the product.

### (3) Product Analysis

The product was separated and qualitatively (and quantitatively) analyzed by gas chromatography directly connected to the fixed-bed reactor. The gas chromatographs used were Agilent 7900 and Techcomp 7900, equipped with a flame ionization detector (FID) (with a methanizer) and a thermal conductivity detector (TCD). The conversion of the raw material and the selectivity of the target product were calculated via the area normalization method, while the carbon balance in the calculation was determined by the internal standard method. The catalyst performance evaluation results of each example and comparative example are listed in Tables 1~6.

**Table 1 Temperature-Programmed Reaction Evaluation Results of Catalyst Ti-Ref**

| Reaction temperature /°C | Propylene conversion /% | Propylene oxide selectivity /% | Propylene oxide yield /mmol g⁻¹h⁻¹ |
|---|---|---|---|
| 380 | 0.5 | 39.2 | 0.1 |
| 420 | 2.2 | 35.1 | 0.4 |
| 460 | 3.1 | 34.8 | 0.6 |
| 500 | 4.2 | 34.1 | 0.8 |
| 540 | 8.4 | 33.8 | 1.6 |

The results in Table 1 demonstrate that at a reaction temperature of 540°C, the catalyst Ti-Ref in Comparative Example 1 can catalyze the direct epoxidation of propylene, achieving a propylene conversion of 8.4% and a propylene oxide selectivity of 33.8%, with a propylene oxide yield of 1.6 mmol g⁻¹h⁻¹. The selectivity of this catalyst for propylene oxide is too low to meet the requirements of industrial production

**Table 2 Temperature-Programmed Reaction Evaluation Results of Catalyst Co-Ref**

| Reaction temperature /°C | Propylene conversion /% | Propylene oxide selectivity /% | Propylene oxide yield /mmol g⁻¹h⁻¹ |
|---|---|---|---|
| 380 | 0.8 | 58.3 | 0.2 |
| 420 | 2.5 | 55.4 | 0.6 |
| 460 | 4.1 | 51.7 | 1.2 |
| 500 | 7.2 | 49.7 | 2.1 |
| 540 | 12.5 | 41.7 | 3.1 |

The results in Table 2 demonstrate that the catalyst Co-Ref in Comparative Example 2 can catalyze the direct epoxidation of propylene, achieving a propylene conversion of 12.5% and a propylene oxide selectivity of 40~50%, with a propylene oxide yield of 3.1 mmol g⁻¹h⁻¹. Although the propylene conversion over this catalyst is higher than that over the catalyst in Comparative Example 1, its propylene oxide selectivity remains relatively low, which still fails to meet the requirements of industrial production.

**Table 3 Temperature-Programmed Reaction Evaluation Results of Catalyst Ti-MCM-41**

| Reaction temperature /°C | Propylene conversion /% | Propylene oxide selectivity /% | Propylene oxide yield /mmol g⁻¹h⁻¹ |
|---|---|---|---|
| 380 | 1.2 | 32.3 | 0.2 |
| 420 | 3.5 | 35.1 | 0.5 |
| 460 | 8.7 | 26.3 | 1.3 |
| 500 | 11.2 | 19.5 | 1.3 |

The MCM-41 zeolite used in Comparative Example 3 is a pure-silica zeolite from the prior art. The results in Table 3 demonstrate that in the direct epoxidation of propylene catalyzed by the catalyst Ti-MCM-41 of Comparative Example 3, the propylene oxide selectivity is relatively low (<35.1%), with a propylene oxide yield of 3.1 mmol g⁻¹h⁻¹, which fails to meet the requirements of industrial production.

**Table 4 Temperature-Programmed Reaction Evaluation Results of Catalyst Ti-A**

| Reaction temperature /°C | Propylene conversion /% | Propylene oxide selectivity /% | Propylene oxide yield /mmol g⁻¹h⁻¹ |
|---|---|---|---|
| 380 | 0.4 | 83.9 | 0.3 |
| 420 | 1.6 | 81.8 | 0.8 |
| 460 | 3.0 | 80.8 | 1.5 |
| 500 | 5.8 | 82.4 | 2.9 |
| 540 | 11.5 | 87.1 | 6.0 |

**Table 5 Temperature-Programmed Reaction Evaluation Results of Catalyst TiAl-A**

| Reaction temperature /°C | Propylene conversion /% | Propylene oxide selectivity /% | Propylene oxide yield /mmol g⁻¹h⁻¹ |
|---|---|---|---|
| 380 | 1.6 | 53.8 | 0.7 |
| 420 | 3.6 | 40.7 | 0.9 |
| 460 | 6.0 | 35.6 | 1.3 |
| 500 | 10.8 | 26.2 | 0.8 |

**Table 6 Temperature-Programmed Reaction Evaluation Results of Catalyst Sn-A**

| Reaction temperature /°C | Propylene conversion /% | Propylene oxide selectivity /% | Propylene oxide yield /mmol g⁻¹h⁻¹ |
|---|---|---|---|
| 380 | 0.4 | 71.9 | 0.3 |
| 420 | 1.7 | 68.4 | 0.7 |
| 460 | 3.3 | 67.1 | 1.4 |
| 500 | 6.1 | 65.4 | 2.4 |
| 540 | 13.0 | 55.1 | 4.3 |

The results in Tables 1~6 show that, under the conditions of a reaction temperature of 540°C, a propylene concentration of 5%, and a total space velocity of 36,000/hour, the epoxidation catalyst of the present disclosure, unlike the catalysts obtained in Comparative Examples 1 and 2, enabled the conversion of propylene to propylene oxide with a considerable conversion rate (11.5%) and a high selectivity (>80%). The propylene oxide yield reached 6.0 mmol g⁻¹h⁻¹, representing an improvement of more than 50% in performance compared to the comparative examples, and is expected to be suitable for large-scale industrial production.

### (4) Reaction Stability Evaluation

**Table 7 Effect of Continuous Reaction Period on Catalytic Activity of Catalyst Ti-A for Propylene Epoxidation**

| Continuous reaction period /h | Propylene conversion /% | Propylene oxide selectivity /% | Propylene oxide yield /mmol g⁻¹h⁻¹ |
|---|---|---|---|
| 50 | 10.8 | 85.0 | 5.6 |
| 100 | 11.5 | 87.2 | 6.0 |
| 150 | 10.9 | 85.6 | 5.6 |
| 200 | 11.0 | 88.4 | 5.7 |
| 250 | 11.1 | 88.5 | 6.1 |
| 300 | 11.1 | 88.5 | 6.1 |
| 350 | 11.1 | 87.6 | 6.0 |
| 400 | 11.2 | 88.3 | 6.0 |

The results in Table 7 and FIG. 4 demonstrate that the catalyst of the present disclosure is able to maintain a stable conversion (~10%) and selectivity (>80%) over a long reaction period (400 hours), making it suitable for industrial production.

Additionally, it should be noted that the various specific technical features described in the above detailed embodiments can be combined in any suitable manner, provided that no contradiction arises. To avoid unnecessary repetition, the present disclosure does not separately describe all possible combinations.

Furthermore, any combinations of the various different embodiments of the present disclosure may be made, provided that such combinations do not contradict the concept of the present disclosure. Such combinations shall also be deemed as part of the content disclosed by the present disclosure.

The foregoing descriptions are merely preferred embodiments of the present disclosure and are not intended to limit the present disclosure in any form. Any simple modifications, equivalent changes, and alterations made to the above embodiments based on the technical essence of the present disclosure shall still fall within the scope of the technical solutions of the present disclosure.

## Claims

1. Use of a catalyst for direct epoxidation of propylene with oxygen to produce propylene oxide, wherein
the catalyst comprises a zeolite support and an active component, the active component being at least one of titanium, tin, molybdenum, or tungsten;
a preparation method of the catalyst comprises the following steps: (1) subjecting a silica-alumina zeolite to a dealumination treatment to obtain a treated zeolite; and (2) mixing the treated zeolite with a metal precursor for introducing the active component, and calcining a resulting mixture in air to obtain the catalyst, wherein
the step (1) comprises the following steps:
(1.1) calcining the silica-alumina zeolite to remove a template, followed by an acid treatment, to obtain a first material;
(1.2) cooling the first material, subjecting it to vacuum filtration and washing, and then drying a filter cake to obtain a second material; and
(1.3) calcining the second material obtained in step (1.2) at a temperature of 450~650°C for 4~12 hours to obtain the treated zeolite, wherein
the silica-alumina zeolite comprises at least one of MOR, MFI, FAU, or BEA zeolite; and
the use comprises:
(a) loading the catalyst into a reactor, performing pretreatment and activation on the catalyst, and then feeding a reaction gas comprising propylene, oxygen, and a balance gas, wherein the balance gas is an inert gas; and
(b) heating the reactor to 200~700°C and allowing the reaction to proceed for 1~3 hours until equilibrium is reached, thereby obtaining propylene oxide.

2. The use according to claim 1, wherein a loading of the active component is 1~8% by mass of the catalyst.

3. The use according to claim 1, wherein the silica-alumina zeolite has a silicon-to-aluminum atomic ratio of 12~50.

4. The use according to claim 1, wherein the dealumination treatment in step (1) is an acid dealumination treatment, and a temperature of the acid dealumination treatment is 60~120°C.

5. The use according to claim 1, wherein the step (2) comprises the following steps:
(2.1) uniformly mixing the treated zeolite obtained in step (1) with a metal precursor to obtain a mixture; and
(2.2) calcining the obtained mixture at 450~650°C for 4~12 hours to obtain the catalyst.

6. The use according to any one of claims 1 to 5, wherein the metal precursor is an organometallic compound precursor, the organometallic compound precursor comprising at least one of an organotitanium precursor, an organotin precursor, an organomolybdenum precursor, or an organotungsten precursor.

7. The use according to any one of claims 1 to 5, wherein in step (a), the pretreatment and activation comprise pretreating and activating the catalyst with air at a temperature of 200~600°C for 1~3 hours.
